# FASCICULE DE BREVET EUROPEEN

(11) **EP 0 836 504 B1**
(45) Date de publication et mention de la délivrance du brevet: **11.10.2000**
(21) Numéro de dépôt: 96924947.3
(22) Date de dépôt: 05.07.1996
(51) Int. Cl.: A61M 29/02

(54) **CATHETER DE DILATATION DU TYPE GUIDE SUR UN FIL**
AUFBLASBARER OVER-THE-WIRE KATHETER
OVER-THE-WIRE INFLATABLE CATHETER

(30) Priorité: 06.07.1995 FR 9508175
(43) Date de publication de la demande: 22.04.1998
(73) Titulaire: Nycomed Amersham Medical Systems S.A., 75644 Paris Cedex 13 (FR)
(72) Inventeur: HILAIRE, Pierre, F-75009 Paris (FR); SALBERT, Philippe, F-95330 Domont (FR)
(74) Mandataire: Boydell, John Christopher
(86) Numéro de dépôt international: FR9601057
(87) Numéro de publication internationale: WO9702067

(56) Documents cités:
- EP-A- 0 441 384
- EP-A- 0 595 308
- WO-A-94/04216
- WO-A-95/09668
- WO-A-95/28197

## Description

La présente invention a pour objet un cathéter de dilatation du type guidé sur un fil ci-après dénommé "over the wire", destiné à être introduit dans un canal corporel tel que notamment un vaisseau sanguin.

L'invention trouve principalement application dans le domaine du traitement des affections des artères coronaires mais peut être également utilisée dans le domaine du traitement des affections d'autres canaux corporels, comme par exemple l'oesophage ou l'urètre.

Ces affections résultent généralement de la présence, sur les parois internes du canal, de dépôts entraînant des rétrécissements ou sténoses de celui-ci.

Dans le traitement de ce type d'affections, un cathéter de dilatation est généralement utilisé pour rétablir la section normale de passage du canal au niveau de la sténose par compression à l'aide d'un ballon (ou ballonnet).

Un guide, habituellement réalisé sous forme d'un fil métallique, est utilisé pour faciliter la progression du cathéter jusqu'à la sténose.

Le fil-guide est généralement plus long que le cathéter (de l'ordre de 20 à 50 cm) pour permettre l'avancement du cathéter à l'intérieur du canal corporel, par glissement le long du fil.

Pour positionner correctement le ballon au niveau de la sténose, il est nécessaire d'amener l'extrémité distale du cathéter au-delà de ladite sténose.

Comme on le comprend, le franchissement de la sténose par l'extrémité distale du cathéter nécessite généralement l'application sur ce dernier d'une poussée.

Cette poussée est exercée par le praticien au niveau de l'extrémité proximale du cathéter.

La transmission de cette poussée jusqu'à l'extrémité distale du cathéter soulève un problème qui n'a pas été résolu jusqu'à ce jour d'une manière satisfaisante.

Le document WO 95/09668 décrit un cathéter de dilatation ayant un stylet effilé qui augmente la rigidité columnaire et la transmission de forces axiales ("poussabilité") du tige du cathéter. Le stylet s'étend distalement au-delà d'un orifice de sortie et se noie dans le tige du cathéter, augmentant par conséquent la rigidité columnaire du tige dans la région de l'orifice de sortie et la poussabilité du cathéter.

Dans ces conditions, la présente invention a pour but de résoudre le problème technique consistant en la fourniture d'un cathéter de dilatation dénommé "over the wire", d'une nouvelle conception, pouvant être réalisée facilement à l'échelle industrielle, dont la mise en oeuvre est aisée, et permettant de transmettre efficacement jusqu'à la partie distale comportant le ballon, la poussée exercée au niveau de la partie proximale.

L'objet de la présente invention est un cathéter de dilatation destiné à être introduit dans un canal corporel tel que notamment un vaisseau sanguin, du type comprenant:
- un corps tubulaire flexible (1) comprenant une partie distale (1c), une partie intermédiaire (1b) et une partie proximale (1a), et comportant:
- une portion déformable radialement formant ballon (2), disposée au niveau de sa partie distale (1c) ;
- un premier conduit interne (5) débouchant à une extrémité à l'intérieur du ballon, de façon étanche, et reliée à l'autre extrémité à une source d'alimentation en fluide pour permettre le gonflage et le dégonflage du ballon (2) ;
- un second conduit interne (6), non communiquant avec ledit premier conduit interne (5), et adapté pour permettre le passage d'un fil-guide (8),
- une âme (11) à haut module élastique reliée fixement par son extrémité proximale au corps (1) et dont l'extrémité distale (13) est noyée dans la paroi définissant ledit second conduit interne (6) au niveau de sa portion intermédiaire (6b),
caractérisé en ce que:
- ledit second conduit interne (6) traverse ledit corps (1) de part en part de façon centrale dans la partie intermédiaire (1b) et dans la partie distale (1c) et de façon excentrée dans la partie proximale (1a);
- ledit corps (1) comprend dans sa partie intermédiaire (1b), un tube externe (14) qui prolonge la portion déformable radialement formant le ballon (2) de la partie distale (1c) et qui entoure coaxialement la portion intermédiaire (6b) du second conduit interne (6) en ménageant un espace intercalaire formant la portion intermédiaire (5b) du premier conduit interne (5);
- ledit corps (1) comprent dans sa partie proximale (1a) un tube à double voies (12) constitué de la portion proximale excentrée (6a) du second conduit interne (6) et d'un canal sensiblement hemicylindrique (5a) formant la portion proximale du premier conduit interne (5) dans lequel est logé le tronçon proximal libre (11a) de ladite âme (11).

Ainsi, l'originalité de la présente invention réside dans l'utilisation d'un élément raidisseur destiné à rigidifier le corps de cathéter et à assurer une transmission sûre et efficace de la poussée exercée au niveau de la partie proximale du cathéter jusqu'au niveau de sa partie distale.

Selon une autre caractéristique, le corps comprend, dans la zone de transition entre sa partie proximale et sa partie intermédiaire, un tube à triple voies, constitué d'une première voie supérieure, prolongeant d'une part le canal hemicylindrique de la partie proximale et débouchant d'autre part dans la portion intermédiaire du premier conduit interne, d'une seconde voie inférieure communiquant de part et d'autre de la zone de transition avec respectivement les portions proximale et intermédiaire du second conduit interne et d'une troisième voie dans laquelle est emprisonné le tronçon correspondant de l'âme métallique.

De préférence, le tube externe est fixé à son extrémité proximale au tube à triple voies et au tube à double voies.

L'âme étant noyée dans la paroi du second conduit interne précité, il n'y a pas de diminution notable de la section de passage du fluide servant au gonflage du ballon. Cette conformation ne réduit donc d'aucune façon le temps de gonflage et de dégonflage du ballon.

Selon une caractéristique particulière, l'âme précitée présente une section transversale qui décroit de son extrémité proximale vers son extrémité distale.

La diminution progressive de la section transversale de l'âme confère donc à l'ensemble du cathéter une progressivité de souplesse en flexion et évite tout risque de cassure or de plicature de celui-ci.

Le cathéter ainsi obtenu présente une rigidité relativement importante en partie proximale, et une certaine souplesse en partie distale, le passage de la partie rigide à la partie plus souple se faisant sans cassure.

En d'autres termes, le cathéter conforme à la présente invention comporte un corps suffisamment rigide pour permettre une bonne transmission de la poussée exercée sur la partie proximale vers la partie distale tout en assurant une flexibilité suffisante au niveau de la partie distale permettant une maniabilité aisée du cathéter notamment au niveau des courbures du canal corporel.

L'expression "à haut module élastique" utilisée dans le cadre de la présente description et des revendications, vise à couvrir tout matériau présentant un module élastique d'au moins 10000 MPa.

Avantageusement, l'âme précitée sera réalisée en un matériau métallique, de préférence en acier.

Selon une autre caractéristique de l'invention, l'âme précitée s'étend dans la partie intermédiaire jusqu'à un point situé en amont et au voisinage immédiat de la portion formant ballon.

Cette conformation particulière permet d'éviter tout risque de perçage du ballon par l'âme précitée tout en assurant la transmission de la poussée jusqu'à la sténose.

L'invention sera mieux comprise, et d'autres buts, caractéristiques et avantages de celle-ci apparaîtront plus clairement à la lecture de la description explicative qui va suivre, faite en référence aux dessins schématiques annexés donnés uniquement à titre d'exemples non limitatifs illustrant un mode de réalisation actuellement préféré de l'invention, et dans lesquels :
- la figure 1 est une vue en coupe longitudinale d'un cathéter dénommé "over the wire" conforme à la présente invention ;
- la figure 2 est une vue en coupe transversale selon la ligne II-II de la figure 1 ;
- la figure 3 est une vue en coupe transversale selon la ligne III-III de la figure 1 ;
- la figure 4 est une vue en coupe transversale selon la ligne IV-IV de la figure 1 ;
- la figure 5 est une vue en coupe transversale selon la ligne V-V de la figure 1 ;

Dans la description qui va suivre, le canal corporel choisi à titre d'exemple est un vaisseau sanguin non représenté, comme en particulier une artère coronaire.

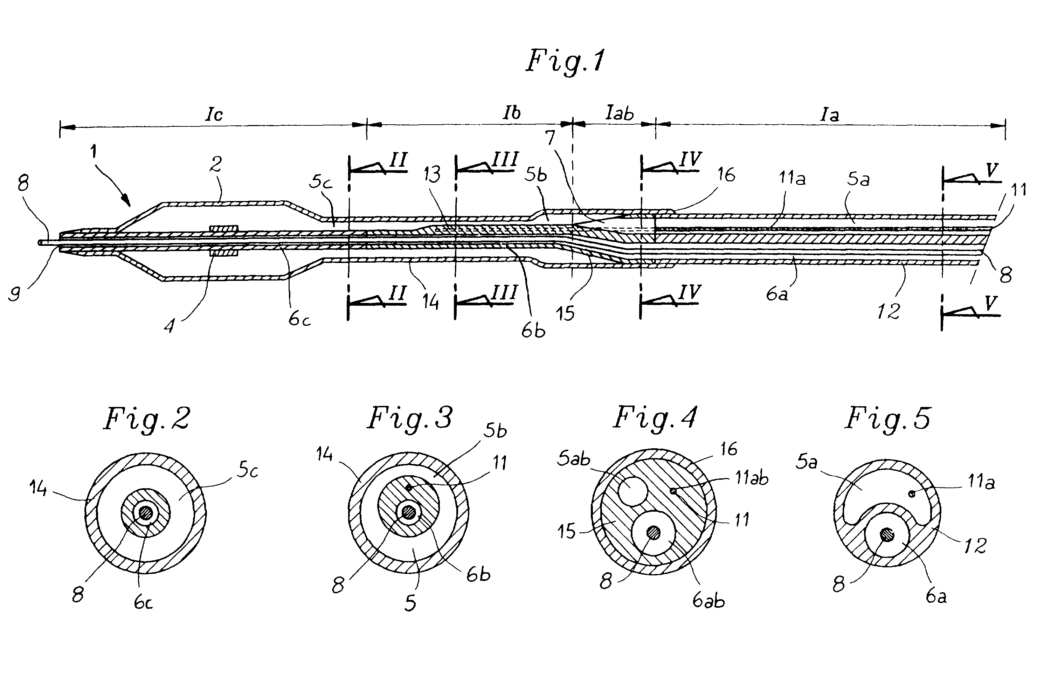

On a donc représenté schématiquement à la figure 1 un cathéter du type dénommé "over the wire" conforme à la présente invention.

Ce cathéter comprend un corps tubulaire flexible 1 comprenant une partie proximale 1a, une partie intermédiaire 1b et une partie distale 1c.

A titre d'exemple, pour un cathéter ayant une longueur totale d'environ 135 cm, la partie proximale 1a peut présenter une longueur d'environ 111 cm, la partie intermédiaire 1b peut présenter une longueur d'environ 21 cm et la partie distale 1c peut présenter une longueur d'environ 3 cm.

De préférence, le corps tubulaire flexible 1 présentera une section transversale sensiblement circulaire et uniforme sur la majeure partie de sa longueur.

Le corps 1 présente au niveau de sa partie distale 1c une portion déformable radialement formant ballon 2 et au niveau de sa partie proximale 1a une portion tubulaire multivoies 12, permettant le raccordement à une source d'alimentation en fluide pour permettre le gonflage et le dégonflage du ballon 2 ainsi que le passage d'un fil guide 8 traversant longitudinalement le corps 1.

Le corps flexible 1 peut être réalisé par exemple en une ou plusieurs matières thermoplastiques semi-rigides choisies parmi les polyéthylènes, les polyamides ou bien encore des copolymères de type PEBAX ® ou HYTREL®.

La portion déformable radialement formant ballon 2 peut être intégrée au corps flexible 1, comme dans l'exemple représenté, ou rapporté sur celui-ci en y étant fixée de façon étanche par des moyens connus comme par exemple par thermosoudure ou à l'aide d'un adhésif. Cette portion pourra être réalisée également en une matière thermoplastique comme notamment un polyamide, un polyéthylène ou bien encore un polyester.

Dans les dessins, on a représenté la partie formant ballon à l'état gonflé.

Pour faciliter le positionnement du ballon 2 au niveau de la sténose (avant gonflage), le corps 1 pourra être équipé d'un moyen de repérage comme par exemple une bague métallique radio-opaque 4 ; un métal tel que l'or, le platine, le tungstène ou leurs alliages pouvant être utilisés pour la réalisation de cette bague radio-opaque.

D'une façon générale, le corps flexible 1 comprend un premier conduit interne 5, s'étendant sensiblement longitudinalement dans la partie distale 1c, et se prolongeant au niveau de la partie intermédiaire 1 b et de la partie proximale 1a.

Le corps flexible 1 comprend également un second conduit interne 6, non communiquant avec le premier conduit interne 5 et traversant également les parties distale 1c, intermédiaire 1b et proximale 1a, la portion distale 5c du premier conduit interne 5 débouche à l'intérieur du ballon 2, de façon étanche, et son extrémité proximale est reliée par l'intermédiaire du tube multivoies 12 à une source d'alimentation en fluide non représentée pour permettre le gonflage et dégonflage du ballon 2.

Dans l'exemple représenté, le tube multivoies 12 est un tube à double voies.

Le second conduit interne 6 est défini par une paroi sensiblement tubulaire qui sera décrite plus en détail ci-après, et est adapté pour permettre le passage du fil-guide 8 débouchant à l'extrémité distale du cathéter par une ouverture 9 prévue à cet effet. Le fil-guide 8 habituellement métallique peut être introduit dans le cathéter par saisie de son extrémité proximale et enfilement dans le second conduit interne 6 à partir de l'embase du cathéter (non représentée sur ce plan) et par avancement jusqu'à l'ouverture distale 9.

Dans le mode de réalisation actuellement préféré et représenté à la figure 1, les conduits internes 5 et 6 s'étendent sensiblement longitudinalement à l'intérieur du corps 1 et sont coaxiaux dans la partie distale 1c et dans au moins une portion de la partie intermédiaire 1b. Les parties proximale 1a et distale 1c sont reliées entre elles de façon étanche par la partie intermédiaire 1 b.

Le corps 1 comporte en outre une âme 11 à haut module élastique qui est reliée fixement par son extrémité proximale au corps 1 par exemple par soudure sur ce dernier et dont l'extrémité distale 13 est noyée dans la paroi définissant la portion intermédiaire 6b du second conduit interne 6.

L'âme 11 présente une section transversale par exemple circulaire, qui décroît de son extrémité proximale vers son extrémité distale 13.

L'extrémité distale 13 se trouve au voisinage immédiat et en amont de la portion formant ballon 2 précitée par rapport au sens d'injection du fluide de gonflage.

Comme on peut le voir sur la figure 1, la paroi qui délimite la portion distale déformable radialement formant le ballon 2 est prolongée par un tube externe 14 qui s'étend sur la partie intermédiaire 1 b. Le tube externe 14 entoure coaxialement la portion intermédiaire centrale 6b du second conduit interne 6 en ménageant ainsi un espace intercalaire qui forme la portion intermédiaire 5b du premier conduit interne 5.

Cette portion intermédiaire 5b ainsi que la portion distale 5c débouchant dans le ballon 2 constituent des conduits internes périphériques.

La partie proximale 1a du corps 1 est constituée par un tube à double voies 12. Ce tube à double voies est constitué comme le montre la figure 5, de la portion proximale 5a du premier conduit interne 5 à l'intérieur duquel est disposé le tronçon proximal libre de l'âme II et de la portion proximale 6a du second conduit interne 6 dans lequel est introduit le fil guide 8. La portion 5a du premier conduit 5 est, par ailleurs, reliée à une source d'alimentation en fluide, non représenté, pour permettre le gonflage et le dégonflage du ballon 2. La portion proximale 6a du second conduit interne 6 est excentrée pour libérer un canal sensiblement hemicylindrique ou de section en croissant formant la portion proximale 5a du premier conduit interne 5.

Le second conduit interne 6 qui est coaxial relativement au tube externe 14 dans les parties distale 1c et intermédiaire 1b, est réalisé en étirant du côté distal un tube multivoies 15.

Dans l'exemple représenté, le tube 15 est un tube à triple voies qui s'étend uniquement dans la zone de transition 1 ab entre les parties intermédiaires 1b et proximale 1a. La section du tube 15 évolue de façon continue d'une extrémité à l'autre de la zone de transition 1ab du fait de l'étirage.

Comme le montre la figure 4, ce tube 15 comporte une première voie supérieure 5ab à section transversale sensiblement circulaire, prolongeant d'une part, le canal hemicylindrique 5a de la partie proximale 1a, débouchant d'autre part, dans la portion intermédiaire 5b du premier conduit interne 5. Le tube 15 comporte également une seconde voie inférieure 6ab communiquant de part et d'autre de la zone de transition 1ab avec respectivement les portions proximale 6a et intermédiaire 6b du second conduit interne 6 ainsi qu'une troisième voie IIab dans laquelle est emprisonné le tronçon correspondant de l'âme métallique II.

Au delà de la zone de transition étirée 1ab, la troisième voie IIab disparaît et l'âme métallique II se trouve noyée dans la paroi de la portion intermédiaire 6b du second conduit interne 6 comme représenté sur les figures 1 et 3. Eventuellement, cette paroi présentera à cet endroit une faible surépaisseur.

Le tube externe 14 est fixé à son extrémité proximale 16 au tube à triple voies 15 et au tube à double voies 12, de préférence par thermosoudure.

Une ouverture 7 est pratiquée au niveau du rétrécissement dans la première voie supérieure 5ab du tube triple voies 15 afin d'assurer ainsi un passage pour le fluide destiné au gonflage et au dégonflage du ballon 2.

En référence à la figure 1, le mode de réalisation de la jonction entre les parties intermédiaire 1b et proximale 1a d'un cathéter de dilatation conforme à la présente invention, se fait par une mise en contact et soudure des tubes 15 et 12 préalablement disposés de façon à mettre en coïncidence les différents conduits l'un par rapport à l'autre. La jonction ainsi obtenue est étanche.

Le fonctionnement et l'utilisation du cathéter de dilatation de type dénommé "over the wire" qui vient d'être décrit est conforme à ceux décrits dans l'état de la technique auquel l'homme de métier pourra se reporter.

D'une façon générale, un cathéter de guidage sera tout d'abord introduit dans le vaisseau du patient.

Ensuite, un cathéter de dilation conforme à la présente invention, de dimension convenable prélablement sélectionnée et un fil de guidage 8 seront introduits dans ce cathéter de guidage par avance initiale du fil de guidage jusqu'à la sténose, puis par avance du cathéter de dilatation jusqu'à ce que le ballon 2 se trouve en vis-à-vis de la sténose.

## Revendications

1. Cathéter de dilatation destiné à être introduit dans un canal corporel tel que notamment un vaisseau sanguin, du type comprenant :
- un corps tubulaire flexible (1) comprenant une partie distale (1c), une partie intermédiaire (1b) et une partie proximale (1a), et comportant :
- une portion déformable radialement formant ballon (2), disposée au niveau de sa partie distale (1c) ;
- un premier conduit interne (5) débouchant à une extrémité à l'intérieur du ballon, de façon étanche, et reliée à l'autre extrémité à une source d'alimentation en fluide pour permettre le gonflage et le dégonflage du ballon (2) ;
- un second conduit interne (6), non communiquant avec ledit premier conduit interne (5), et adapté pour permettre le passage d'un fil-guide (8),
- une âme (11) à haut module élastique reliée fixement par son extrémité proximale au corps (1) et dont l'extrémité distale (13) est noyée dans la paroi définissant ledit second conduit interne (6) au niveau de sa portion intermédiaire (6b),
caractérisé en ce que :
- ledit second conduit interne (6) traverse ledit corps (1) de part en part de façon centrale dans la partie intermédiaire (1b) et dans la partie distale (1c) et de façon excentrée dans la partie proximale (1a) ;
- ledit corps (1) comprend dans sa partie intermédiaire (1b), un tube externe (14) qui prolonge la portion déformable radialement formant le ballon (2) de la partie distale (1c) et qui entoure coaxialement la portion intermédiaire (6b) du second conduit interne (6) en ménageant un espace intercalaire formant la portion intermédiaire (5b) du premier conduit interne (5) ;
- ledit corps (1) comprend dans sa partie proximale (1a) un tube à double voies (12) constitué de la portion proximale excentrée (6a) du second conduit interne (6) et d'un canal sensiblement hemicylindrique (5a) formant la portion proximale du premier conduit interne (5) dans lequel est logé le tronçon proximal libre (11a) de ladite âme (11).

2. Cathéter selon la revendication 1, caractérisé en ce que le corps (1) comprend, dans la zone de transition (1ab) entre sa partie proximale (1a) et sa partie intermédiaire (1b), un tube (15) à triple voies, constitué :
- d'une première voie supérieure (5ab), prolongeant d'une part le canal hemicylindrique (5a) de la partie proximale (1a) et débouchant d'autre part dans la portion intermédiaire (5b) du premier conduit interne (5),
- d'une seconde voie inférieure (6ab) communiquant de part et d'autre de la zone de transition (1ab) avec respectivement les portions proximale (6a) et intermédiaire (6b) du second conduit interne (6) ; et
- d'une troisième voie (11ab) dans laquelle est emprisonnée le tronçon correspondant de l'âme métallique (11).

3. Cathéter selon les revendications 1 et 2, caractérisé en ce que le tube externe (14) est fixé à son extrémité proximale (16) au tube à triple voies (15) et au tube à double voies (12).

4. Cathéter de dilatation selon l'une des revendications précédentes, caractérisé en ce que l'âme (11) précitée présente une section transversale qui décroît de son extrémité proximale vers son extrémité distale (13).

5. Cathéter de dilatation selon l'une des revendications précédentes, caractérisé en ce que l'âme (11) précitée est métallique, notamment en acier.

6. Cathéter de dilatation selon l'une des revendications précédentes, caractérisé en ce que l'âme (11) précitée s'étend dans la partie intermédiaire (1b) jusqu'à un point situé au voisinage immédiat de la portion formant ballon.

## Patentansprüche

1. Dilatationskatheter zum Einführen in einen Körperkanal, insbesondere ein Blutgefäß, von der Art umfassend:
- einen biegsamen oder flexiblen röhrenförmigen Körper (1), der ein distales Teilstück (1c), ein mittleres Teilstück (1b) und ein proximales Teilstück (1a) umfasst und aufweist:
- einen in Höhe seines distalen Teilstücks (1c) angeordneten radial verformbaren Teil, der einen Ballon (2) bildet;
- einen ersten inneren Kanal (5), der an einem Ende in dichter Weise ins Innere des Ballon mündet und am anderen Ende mit einer Fluidversorgungsquelle verbunden ist, um das Aufpumpen und das Entleeren des Ballons (2) zu ermöglichen;
- einen zweiten inneren Kanal (6), der nicht mit dem besagten ersten inneren Kanal (5) kommuniziert und angepasst ist, um den Hindurchtritt eines Führungsdrahtes (8) zu gestatten,
- eine Seele (11) mit hohem Elastizitätsmodul, die durch ihr proximales Ende fest mit dem Körper (1) verbunden ist und deren distales Ende (13) in die Wand eingebettet ist, die den zweiten inneren Kanal (6) in Höhe seines mittleren Teils (6b) begrenzt,
dadurch gekennzeichnet, dass:
- der besagte zweite innere Kanal (6) den besagten Körper (1) von einer Seite zur anderen durchquert, in mittiger Weise im mittleren Teilstück (1b) und im distalen Teilstück (1c) sowie in exzentrischer Weise im proximalen Teilstück (1a);
- der besagte Körper (1) in seinem mittleren Teilstück (1b) ein äußeres Rohr (14) umfasst, das den den Ballon (2) bildenden radial verformbaren Teil des distalen Teilstücks (1c) verlängert und das den mittleren Teil (6b) des zweiten inneren Kanals (6) unter Aussparung eines den mittleren Teil (5b) des ersten inneren Kanals (5) bildenden Zwischenraums koaxial umgibt;
- der besagte Körper (1) in seinem proximalen Teilstück (1a) ein Zweiwegerohr (12) umfasst, das aus dem exzentrisch angeordneten proximalen Teil (6a) des zweiten inneren Kanals (6) und aus einem den proximalen Teil des ersten inneren Kanals (5) bildenden, im Wesentlichen halbzylindrischen Kanal (5a) besteht, in dem das freie proximale Stück (11a) der besagten Seele (11) untergebracht ist.

2. Katheter nach Anspruch 1, dadurch gekennzeichnet, dass der Körper (1) in der Übergangszone (1ab) zwischen seinem proximalen Teilstück (1a) und seinem mittleren Teilstück (1b) ein Dreiwegerohr (15) umfasst, bestehend:
- aus einem ersten oberen Weg (5ab), der auf einer Seite den halbzylindrischen Kanal (5a) des proximalen Teilstücks (1a) verlängert und auf der anderen Seite in den mittleren Teil (5b) des ersten inneren Kanals (5) mündet,
- aus einem zweiten unteren Weg (6ab), der auf beiden Seiten der Übergangszone (1ab) mit dem proximalen Teil (6a) bzw. dem mittleren Teil (6b) des zweiten inneren Kanals (6) kommuniziert; und
- aus einem dritten Weg (11ab), in dem das entsprechende Stück der metallischen Seele (11) eingeschlossen ist.

3. Katheter nach den Ansprüchen 1 und 2, dadurch gekennzeichnet, dass das äußere Rohr (14) an seinem proximalen Ende (16) am Dreiwegerohr (15) und am Zweiwegerohr (12) befestigt ist.

4. Dilatationskatheter nach einem der vorangehenden Ansprüche, dadurch gekennzeichnet, dass die vorgenannte Seele (11) einen Querschnitt aufweist, der von ihrem proximalen Ende zu ihrem distalen Ende (13) hin abnimmt.

5. Dilatationskatheter nach einem der vorangehenden Ansprüche, dadurch gekennzeichnet, dass die vorgenannte Seele (11) metallisch ist, insbesondere aus Stahl.

6. Dilatationskatheter nach einem der vorangehenden Ansprüche, dadurch gekennzeichnet, dass sich die vorgenannte Seele (11) im mittleren Teilstück (1b) bis zum einem Punkt erstreckt, der sich in unmittelbarer Nachbarschaft des den Ballon bildenden Teils befindet.

## Claims

1. Dilatation catheter for introduction into a body canal, such as in particular a blood vessel, of the type comprising:
- a flexible tubular body (1) comprising a distal part (1c), an intermediate part (1b) and a proximal part (1a), and having:
- a radially deformable portion forming a balloon (2), disposed at the level of its distal part (1c);
- a first inner duct (5) issuing at one end inside the balloon, in liquid-tight manner, and connected at the other end to a fluid supply source in order to enable inflating and deflating of the balloon (2);
- a second inner duct (6), which does not communicate with said first inner duct (5) and is adapted to allow the passage of a guide-wire (8),
- a core (11) having a high modulus of elasticity and being permanently joined to the body (1) by its proximal end and of which the distal end (13) is embedded in the wall defining said second inner duct (6) at the level of its intermediate portion (6b),
characterised in that:
- said second inner duct (6) passes right through said body (1) centrally in the intermediate part (1b) and in the distal part (1c) and eccentrically in the proximal part (1a);
- said body (1) comprises in its intermediate part (1b) an outer tube (14) which extends the radially deformable portion, forming the balloon (2), of the distal part (1c) and which coaxially surrounds the intermediate portion (6b) of the second inner duct (6) while providing an intercalated space which forms the intermediate portion (5b) of the first inner duct (5);
- said body (1) comprises in its proximal part (1a) a two-channel tube (12) consisting of the eccentric proximal portion (6a) of the second inner duct (6) and of a substantially semi-cylindrical channel (5a) forming the proximal portion of the first inner duct (5), inside which the free proximal section (11a) of said core (11) is contained.

2. Catheter according to claim 1, characterised in that the body (1) comprises, in the transitional zone (1ab) between its proximal part (1a) and its intermediate part (1b), a three-channel tube (15) consisting of:
- a first upper channel (5ab) extending, on the one hand, the semi-cylindrical channel (5a) of the proximal part (1a) and, on the other hand, issuing in the intermediate portion (5b) of the first inner duct (5),
- a second lower channel (6ab) communicating on each side of the transitional zone (1ab) with, respectively, the proximal portion (6a) and the intermediate portion (6b) of the second inner duct (6); and
- a third channel (11ab) inside which the corresponding section of the metallic core (11) is confined.

3. Catheter according to claims 1 and 2, characterised in that the outer tube (14) is fixed at its proximal end (16) to the three-channel tube (15) and to the two-channel tube (12).

4. Dilatation catheter according to any one of the preceding claims, characterised in that the above-mentioned core (11) has a cross section which reduces from its proximal end to it distal end (13).

5. Dilatation catheter according to any one of the preceding claims, characterised in that the above-mentioned core (11) is of metal, in particular steel.

6. Dilatation catheter according to any one of the preceding claims, characterised in that the above-mentioned core (11) extends in the intermediate part (1b) up to a point situated in the immediate vicinity of the balloon-forming portion.
